# EUROPEAN PATENT SPECIFICATION

(11) **EP 0 410 893 B1**
(45) Date of publication and mention of the grant of the patent: **04.10.1995**
(21) Application number: 90402160.7
(22) Date of filing: 26.07.1990
(51) Int. Cl.: G01N 33/68, G01N 33/553, G01N 33/571, G01N 33/564

(54) **Determination and detection of antibody and its immunoglobulin class**
Nachweis eines Antikörpers und dessen Immunglobulin-Klasse
Détection de l'anticorps et sa classe d'immunoglobuline

(30) Priority: 28.07.1989 JP 195968/89; 20.06.1990 JP 162056/90
(43) Date of publication of application: 30.01.1991
(73) Proprietor: Mitsubishi Chemical Corporation, Chiyoda-ku Tokyo (JP)
(72) Inventor: Ito, Michio, Yokohama-shi, Kanagawa-ken (JP); Ogura, Minoru, Yokohama-shi, Kanagawa-ken (JP); Kohno, Hideki, Kawasaki-shi, Kanagawa-ken (JP)
(74) Representative: Gutmann, Ernest

(56) References cited:
- EP-A- 0 291 389
- WO-A-89/01161
- FR-A- 2 531 223
- WORLD PATENTS INDEX LATEST, Week 8543; Derwent Publications Ltd., London (GB); AN 85-265974

## Description

### FIELD OF THE INVENTION

This invention relates to a method for determining an antibody in a biological fluid sample against a specific antigen and identifying the immunoglobulin class of the antibody. Particularly, it relates to a method for determining and detecting an antibody, which may advantageously be used as a clinical test such as serum test for the diagnosis or differential diagnosis of infectious disease or an autoimmune disease in the field of medicinal chemistry.

### BACKGROUNG OF THE INVENTION

In an infectious disease or an autoimmune disease, an antibody against a specific antigen which is inherent in the disease appears in the serum of a patient. In the conventional clinical test, what has simply been examined is whether or not the serum contains the antibody. But in recent years, determination of an amount of the antibody and classification of its immunoglobulin class (IgG, IgA, IgM, IgD and IgE in the case of human beings) has attracted attention in order to inspect the cause of the disease or morbid state precisely.

For example, in the case of the conventional diagnosis of an infectious disease, the presence of an antibody against a pathogen of the infectious disease shows only the past history of the same infectious disease. However, if a change in the amount of the antibody and the class of immunoglobulin are ascertained, information concerning the time of infection or the advanced stage of the disease can be obtained. More specifically, as introduced in the journal of "Japan Clinics", Vol. 43, Autumn Special Issue, Second volume, page 27 (1985) with the title of "Interpretation of assay results of virus antibody", the detection of IgM antibody is required for the diagnosis of rubella.

Even if the immunoglobulins to be assayed have the same antibody class, this is a case wherein it is examined which kind of an antigen they react with among many kinds of antigens. For example, in the allergic diagnosis the most important immunoglobulin class is IgE, and the RAST method (radioallergen adsorbent test) has been practiced as an available test method, wherein a kind of allergen as an etiogenic substance is identified and an amount of IgE reacting with the allergen is measured.

In this field of the art, RIA and EIA are methods which have taken the leadership as quantitative assay methods. RIA and EIA are not only capable of carrying out a quantitative determination with high sensitivity, but are also capable of identifying the immunoglobulin class to be assayed, so that they are significant in the diagnosis. However, these methods have disadvantages that they require separation and washing of the antibody reacted with the antigen and that the operation is complicated and takes a lot of time. Also, since many operations in which the sample should be treated many times, such as separating and washing, are required, a danger of infection from the sample cannot be ignored. In addition, RIA involves many difficulties in handling, such as problems in radioactive waste and the necessity for specific equipment. EIA has problems in that reaction time and temperature must be strictly controlled and that it is likely affected by an obstruction reaction, since an enzyme is used as a labelling substance.

Other assay techniques of the antibody in the field of the diagnotic methods, there can be mentioned passive blood cell agglomeration and latex agglomeration, in addition to the above RIA and EIA. However, these methods have disadvantages in that they are limited only to a qualitative diagnosis, that the immunoglobulin class cannot be identified and that the sensitivity of detection is relatively low. For overcoming such problems partially, there is disclosed a method for detecting the immunoglobulin class of the antibody using antigen-carrying magnetic particles and blood cells carrying antiglobulin antibody (Japanese Patent Application Laid-Open (KOKAI) No. 177265/1985).

For identifying the class of the antibody to a specific antigen and determining the amount thereof by using the above agglomeration method, it is necessary to measure the amount of agglomeration produced by the reaction between the antigen-carrying particles and the antigen-reactive antibody in the sample. In this event, an agglomeration of antibodies may sometimes occur due to the antigen-carrying particle only. Another agglomeration of the particles carrying the anti-antibody which is specific to the immunoglobulin class of said antibody, may possibly occur as well due to immunoglobulins which are non-bindable to said antigen and present in the sample serum in large amounts. Therefore, there still remain several problems without a solution. For example, undesirable steps which contain the most troublesome operations such as washing and resuspending are still required in the subsequent steps. Indicatively, the antigen-carrying particles should first be reacted with a sample, and the reaction mixture is then separated and washed to remove the immunoglobulins which are non-bindable to said antigen, and then insoluble particles carrying the substance specific to the immunoglobulin class of the antibody are reacted therewith. Also, the time period for completing the assay is relatively long. Detection sensitivity is, in addition, inferior to that of RIA or EIA.

In the publication of WO 89/01161, there is disclosed a method in which magnetic material-containing particles carrying a monoclonal antibody thereon and an antigen in a specimen are reacted followed by reacting with insoluble particles carrying an antibody thereon but containing no magnetic particles; then applying a magnetic field thereto to separate unreacted magnetic material-containing particles from agglomerated bulk comprising said two kinds of particles and the antigen, and measuring the amount of the remaining antibody-carrying insoluble particles. In this method the amount of antigen in the specimen can be determined from a degree of decrease in turbidity. However, there is no description concerning the identification of the immunoglobulin class of the antibody.

Particles for use in biological applications are described, for instance in EP 0 291 389, which discloses polymeric particles comprising, implanted at their surface, amphipathic molecules carrying ionic or reactive groups.

French Patent n° 2 531 223 discloses a procedure for immunologically measuring the total quantity of two or more substances in which two or more antibodies or antigens are utilized as insoluble antigens or insoluble antibodies. The methods of agglutination reactions, sandwich methods, competition and immunometric assays are described. Thus, it is desirable to develop an assay method with high sensitivity, which can determine the amount of antibody, can identify the immunoglobulin class of the same and can be completed within a short time frame.

### SUMMARY OF THE INVENTION

The inventors of the present invention have found that the reaction between antigen-carrying substrate particles and the antigen-reactive antibody in a sample can quantitatively be determined and detected not directly by estimating an amount of agglomeration produced by the reaction in the sample, but indirectly by estimating the remaining unreacted components in the reaction mixture from which the agglomeration is removed, and that the agglomeration can be easily separated from the unreacted components by using a magnetic material-containig substrate carrying a substance which is particularly reactive to a specific immunoglobulin class. As the result of further intensive studies, they have additionally found that separating and washing may suprisingly be omitted after the reaction between the antigen and the antibody in the sample, which are essential in the conventional methods, and that a high sensitivity superior to that of EIA may be obtained within a shorter period of time than in the conventional methods.

An object of the present invention is to provide a safe method of determining and detecting an antibody, which has a high sensitivity superior to that of RIA or EIA and makes it possible to determine the amount of antibody and to identify its immunoglobulin class without carrying out complicated separating and washing operations.

In accordance with the invention, a method is provided for the determination and detection of an antibody in a biological fluid sample against a specific antigen and of its immunoglobulin class, comprising the steps of:
(a) providing a first reagent of insoluble substrate particles carrying the antigen on the surface thereof and a second reagent of insoluble substrate particles carrying on the surface thereof a substance particularly reactive to a specific immunogloblin class, the latter reagent containing a magnetic material;
(b) reacting the sample with both of the reagents in a reaction system;
(c) agglomerating the antigen-carrying particles together with the magnetic material-containing particles through the antibody;
(d) separating locally in the reaction system both the unreacted magnetic material-containing particles and the resultant agglomerate by applying a magnetic field to the reaction system; and
(e) visually or spectrophotometrically estimating the amount of the antigen-carrying particles which remain unreacted in the reaction system.

### BRIEF DESCRIPTION OF THE DRAWINGS

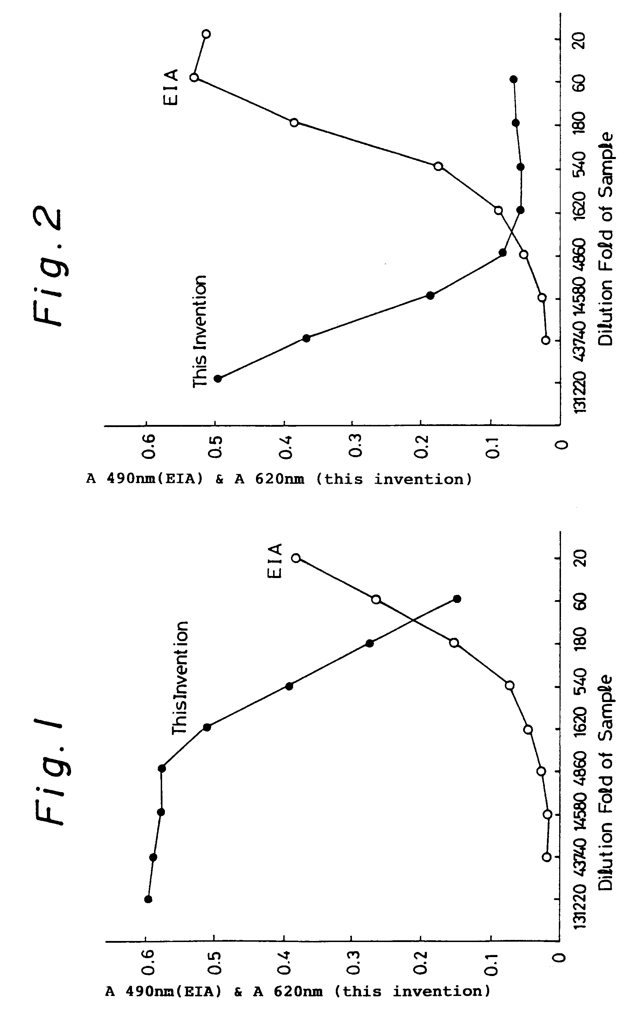

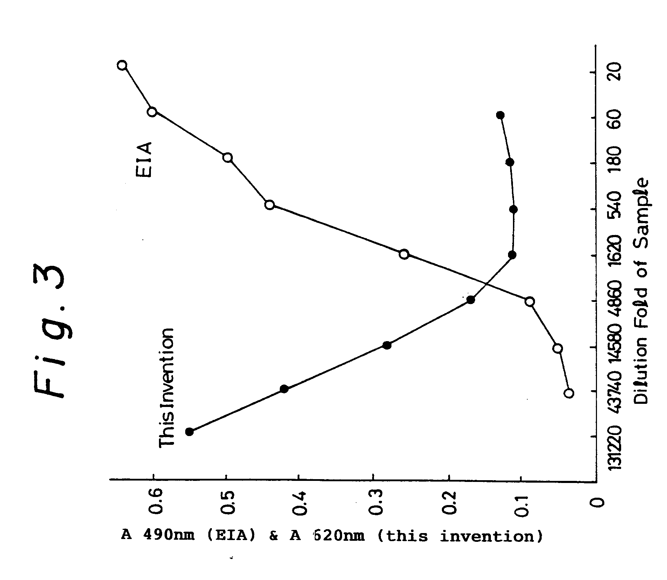

Fig. 1 is a drawing which shows the reactivity of IgG class rheumatoid factor with a dilution series of a serum sample from a rheumatoid patient according to the present invention (solid circle) and EIA method (white circle), in which the reactivity is shown by absorbance. Figs. 2 and 3 are drawings showing the reactivity of IgA and IgM class rheumatoid factors with the same samples as in Fig. 1, respectively.

### DESCRIPTION OF THE PREFERRED EMBODIMENTS

The term "antigen", referred to in the present invention, includes a single substance or plural substances or a mixture thereof, which are selected for a specific object such as diagnosis, among all the substances having the ability of causing the production of an antibody in a human being or an animal. For example, in case of diagnosing an infectious disease, there can be mentioned virus, bacteria, or specific proteins and saccharide chains which constitute those virus and bacteria.

As the "insoluble substrate particles" of the first reagent for carrying the antigen, there may be used, for example, cells such as erythrocytes, gelatin particles, microcapsules such as liposomes, organic polymer substances such as latex particles of polyvinyltoluene and polystyrene, inorganic fine particles such as carbon black, or colloidal particles of various metals or metal compounds, but those having excellent dispersibility in a reaction medium and hardly sedimented by themselves are preferred for the spectrophotometric determination.

A "substance particularly reactive to a specific immunoglobulin class" means a substance having the ability of binding to an antibody by selectively recognizing characteristics of the antibody molecule; for instance, an antibody against IgG, IgA, IgM, IgD, IgE or L(light)-chains thereof; protein A; and C1_{q} which is a kind of complement component . However, the "substance" does not include those which are bindable to the antibody through an antibody activity possessed by said immunoglobulin itself, i.e., a substance which reacts as an antigen with said immunoglobulin.

As the "insoluble substrate particles" of the second reagent containing the magnetic material, there may be used polysaccharides such as agarose, dextrane and carboxymethyl cellulose, proteins such as gelatin and polymerized albumin and protein derivatives, and more preferably synthetic polymers obtained by polymerization of aromatic vinyl compounds such as styrene and divinylbenzene and/or methacrylate derivatives.

As the "magnetic material" which said second reagent particles should contain, preferred are iron, magnetic iron oxide such as tri-iron tetroxide, and mixtures thereof or alloy with other metal or metal oxides. The magnetic material advantageously has no residual magnetization. Also an average particle size of the magnetic material is preferably 1-20 nm (10 to 200 Å) in diameter. The amount of these materials which the second reagent particles contain is, preferably at least 5 % by weight of the second particles, more preferably, 15 to 65 % by weight.

An average particle size of the first reagent particles and that of the second reagent particles are 0.1 to 10 µm in diameter, preferably 0.2 to 3 µm in diameter. For a combination of both particles, there may be used a combination of the first reagent particles having an average particle size of 0.5 to 3 µm and the second reagent particles having an average particle size of 0.2 to 2 µm, preferably, a combination of the first reagent particles having an average particle size of 1 to 2.5 µm and the second reagent particles having an average particle size of 0.5 to 1.5 µm. With respect to the average particle sizes of such particles, if the average particle size of the first reagent particles is too small, the surface area per unit weight becomes so large that the amount of the carried antigen becomes also large, whereby agglomeration between the first reagent particles will likely occur. When the determination of this invention is carried out using a light of a wavelength of 600 to 1000 nm, which is a range less affected by the sample component, this agglomeration reaction is undesirable since it increases turbidity and causes lowering in sensitivity. Such agglomeration reaction may also occur when the average particle size of the second reagent particles is too large. Furthermore, in case that the average particle size of the first reagent particles is too large, natural sedimentation of the particles are promoted, and in case that the average particle size of the second reagent particles is too small, separation by a magnetic field takes a lot of time. Accordingly, these cases are not practical.

For carrying the antigen or the substance particularly reactive to the specific immunoglobulin class on the substrate particles, either physical adsorption or chemical adsorption based on covalent bonding of the functional groups may be used.

The ratio of amounts of the first or second insoluble substrate particles and the antigen or substance to be carried thereon is not particularly limited, but when a 5 to 200-fold amount of the substrate particles in weight ratio to the antigen or substance are used, good results can in general be obtained.

In the present invention a sample solution which possibly contains an antibody is mixed and reacted with the insoluble substrate particles carrying the antigen thereon and the magnetic material-containing particles carrying the substance particularly reactive to the specific immunoglobulin class in a reaction system. Stirring at an initial stage of the reaction should be carried out sufficiently, but after mixing homogenously, the reaction may proceed without stirring. The pH in the reaction is preferably 5 to 10, more preferably 7 to 9 as in the general immunochemical reaction. The temperature in the reaction is in the range of 2 to 50 °C, desirably room temperature to 37-40 °C. The reaction time may be optional varying from a very short reaction period to one day and night, but in view of the sensitivity and operability, it is usually set within the range of 5 to 60 minutes. These reaction conditions are the same in the subsequent procedural steps.

The desired pH range is usually maintained by a buffer. As the buffer, for example, a phosphate and tris(hydroxymethyl)aminomethane can typically be used, but in almost all the buffers usually from neutral to weak alkaline can be used. In general, salts such as sodium chloride and proteins such as bovine serum albumin are added in order to avoid non-specific reactions.

When a sample is mixed with the antigen-carrying insoluble substrate particles and the magnetic material-containing particles carrying the substance which are particularly reactive to the certain immunoglubulin class, an antibody in the sample reacts with the antigen at the surface of the substrate particles and further the antibody bound to the antigen binds to the substance carried on the magnetic material-containing particles to cause agglomeration between the insoluble substrate particles and the magnetic material-containing particles. In this case, either binding between the antigen carried on the insoluble substrate particles and the antibody in the sample or binding between the antibody in the sample and the substance carried on the magnetic material-containing particles may be preceded, or simultaneously proceeded. However, it is more preferred to react the antibody in the sample first with the antigen-carrying insoluble substrate and then with the magnetic material-containing particles carrying the substance particularly reactive to the certain immunoglobulin class. This is because improvement in sensitivity can be more expected than the reverse case due to advancement in reaction-efficiency of the magnetic material-containing particles.

A ratio of the above antigen-carrying insoluble substrate particles and the magnetic material-containing particles carrying the substance particularly reactive to the specific immunoglobulin class to be used may be selected from the range of 1 : 20 to 20 : 1, preferably 1 : 4 to 4 : 1. If either of the particles deviates from this range, the sensitivity will be lowered since the agglomeration of the antigen-carrying insoluble substrate particles with each other or that of the magnetic material-containing particles with each other will possibly occur.

As the reaction between the antigen-carrying insoluble substrate and the antibody in the sample, the following two circumstances can be considered :
1) one molecule of the antibody binds to two particles, to cause agglomeration.
2) one molecule of the antibody reacts with only one particle, so that no agglomeration between particles occurs.

In a conventional assay method on the basis of the latex agglomeration reaction, it is preferred that the reaction of the circumstance 2) occurs in a smaller quantity, since the result of the above circumstance 1) is detected. In the present invention, however, less of circumstance 1) and more of circumstance 2) is desired. This is because reaction 1) can occur regardless of the class of the antibody and it affects the spectrophotometric determination, whereby the precision in the determination of each antibody class is lowered.

As the specific manner to cause both less the reaction 1) and more the reaction 2), there may be mentioned as follows:
(1) to carry the antibody on the first reagent particles with high density by physical adsorption or covalent bonding.
(2) to use the first reagent particles having the average size of about 1 µm or more, preferably 1 to 2.5 µm, which is larger than that of the insoluble substrate particles having the average size of about 0.1 to 0.8 µm conventionally employed, thereby increasing the density of the antigens per unit surface of the first reagent particle.
(3) to lower the concentration of the first reagent particles in the reaction mixture or use the first reagent particles having the average particle size of about 1 µm or more, preferably 1 to 2.5 µm and high specific gravity which does not cause natural sedimentation, thereby decreasing the collision of the particles,i.e.,collision probability between them.

However, the aforesaid factors cause a lowering in the precision which are acceptable so long as they do not cause a practical problem. In fact, reaction 1) can be relatively restrained, or even if reaction 1) occurs, the effects to the spectrophotometric determination results can be minimized by optionally selecting the amount of the antigen-carrying insoluble particles, the amount of the carried antigen, and the size of the particle. For instance, the average particle size of the antigen-carrying insoluble substrate particles is selected to be 1 to 2.5 µm, the average particle size of the magnetic material-containing particles is selected to be 0.5 to 1.5 µm and the ratio of the insoluble antigen-carrying substrate particles and the magnetic material-containing particles to be used is further selected to be 1 : 0.8 to 2.

Accordingly, the present invention is not limited only to circumstance 2) in the reaction but includes both circumstances 1) and 2).

In the step of applying a magnetic field to the reaction system, it is preferred to utilize a magnetic strength and a reaction system which enables both the magnetic material-containing particles and the agglomeration containing the magnetic material-containing particles to be easily separated in 5 to 20 minutes. A too short time period required for the separation usually leads to low sensitivity and reproducibility, while a too long time period leads to undesirable operability. For this reason, the reaction system with a relatively small size can be handled easily. For example, a microplate is employed for the system. A size of each well in the microplate such as a 96-hole microplate is small. Thus, when small magnets are placed at the spaces between each of the wells in the 96-hole microplate, a determination can be easily carried out by using a microplate reader as in EIA utilizing the microplates. Therefore, the microplate is suitable for the present invention. As the magnet for applying the magnetic field, a permanent magnet or an electromagnet can be used.

When the magnetic material-containing particles are separated by means of the magnetic field, the antigen-carrying substrate particles agglomerated with the magnetic material-containing particles are also separated. Consequently, a degree of reaction of the antigen and antibody in the sample can be easily estimated by detecting the turbidity or the amount of the unreacted substrate particles suspended in the reaction solution after the separation. That is, the stronger the reaction between said antigen and antibody in the sample, the less the turbidity (absorbance) becomes.

At this stage, it may occur that a part of the magnetic material-containing particles remains without being separated, and is detected together with the innreacted antigen-carrying substrate particles. However, this is acceptable so long as it causes no practical problem.

An inspecting means of the turbidity includes, most simply, a visual observation with eyes under illumination on a black background, thereby roughly estimating the residual amounts of the antigen-carrying insoluble substrate particles in the reaction solution depending on the degree of the turbidity. Use of various colorimeters or turbidimeters makes a precise quantitative determination possible. As the wavelength for determination, a wavelength corresponding to a visible light or near infrared ray, preferably 600 to 1100 nm can be used. Moreover, by a flow cytometry method using a laser beam, the number of the residual particles may be directly counted.

For the determination, a calibration curve of an antibody should be previously obtained by using a sample having an pre-determined antibody concentration or a sample having a standard amount of an antibody, and plotting the resulting values to the concentration of the antibody in the sample. Thus, from the measured value of an unknown sample in concentration, the concentration of said antibody can be obtained.

### EXAMPLES

The present invention will be hereinafter explained in more detail by referring to the Examples, but it should be noted that the scope of the present invention is by no means limited thereto.

### Example 1

### Determination of anti-cardiolipin antibody

### Preparation of reagents:

### 1) Preparation of a suspension of carbon powder carrying cardiolipin as an antigen thereon.

In 4 ml of ethanol was suspended and dispersed 10 mg of carbon powder (Thermal Carbon MT, produced by MITSUBISHI KASEI CORPORATION, particle diameter: 0.3 µm) . The carbon powder was frequently subjected to an ultrasonic wave treatment to disperse it (same in the subsquent procedures, if necessary), since it is likely to sedimente and agglomerate. The carbon powder was then sedimented by centrifugation and the supernatant was removed. Then, 4 ml of ethanol solution containing 0.03 % of cardiolipin, 0.03 % of lecithin and 0.9 % of cholesterol was added to the sediment followed by dispersion, and the dispersion was further stirred for 30 minutes to homogenize it.

Subsequently, to 3.2 ml of 0.1 M phosphate bufferred saline solution, hereinafter referred to as "PBS", pH 7.4, vigorously stirred, was promptly added dropwise the whole amount of the carbon powder dispersion, and 16.4 ml of the PBS solution was added thereto followed by continuting the vigorous stirring for further 10 minutes to adsorb fat soluble components including the antigen on the surface of the carbon powder. After completion of the adsorption, the dispersion was centrifuged and the supernatant was removed. Then, 20 ml of phosphate buffer, pH 7.4, containing 10 % of choline chloride was added to the sediment and then redispersed to obtain a desired reagent. The carbon powder suspension thus obtained has the same sensitivity as or more sensitivity than a commercially available product as a reagent for RPR method (a method of detecting anticardiolipin antibody by a visual inspection of carbon powder agglomeration), which is one of the assays for syphilis.

### 2) Preparation of a magnetic material-containing latex particles carrying anti-IgG, IgM antibody F(ab')₂ thereon;

An antiserum obtained by immunizing rabbit against human IgG or human IgM was adsorbed onto Bence Jones protein (χ and λ) to separate a specific antiserum to H-chain of IgG and IgM. Each fraction of anti-IgG and anti-IgM antibodies was collected from the resultant antiserum by a conventional method, and then digested with pepsin and subject to molecular sieve column chromatography to obtain F(ab')₂.

On the other hand, 1 ml of a magnetic material-containing latex (Estapor® SML266, particle size: 0.7 µm, 10 %) produced by Rhone Poulenc Chimie was sufficiently mixed with 19 ml of distilled water followed by centrifugation (10000 rpm, 10 minutes), and then the supernatant was removed to obtain a washed latex pellet. To the pellet was added and dispersed an antibody solution in which was dissolved 4 mg of anti-IgG or anti-IgM antibody F(ab')₂ previously prepared in 10 ml of 0.1 M Tris-hydrochloric acid buffer, pH 8, hereinafter referred to as "Tris buffer". The dispersion was further stirred for one hour to carry F(ab')₂ on the surface of the magnetic material-containing latex. The dispersion was centrifuged again to remove the supernatant followed by re-dispersing, suspending and stabilizing with 10 ml of the Tris buffer containing 0.3 % of bovine serum albumin. The resultant latex was further centrifuged and suspended in 10 ml of Tris buffer containing 0.05 % of sodium azide, and preserved at 4 to 10 °C.

### Procedure of the determination:

Each syphilis patient serum, which is positive (detection limit dilution: 16-fold) by the RPR method, and normal serum, which is negative by the same method , was diluted with 0.1 M Tris hydrochloric acid buffer, pH 8.2, containing 0.1 % of bovine serum albumin and 0.9 % of salt, hereinafter referred to as "TBS buffer", to from 20-fold to 320-fold stepwise by a 2-fold dilution, to prepare a diluted series of sera. Each of the samples of the diluted series and TBS buffer as a control was apportioned to two wells in a 96-hole microplate in an amount of 100 µl. After each 50 µl of the cardiolipin antigen-carrying carbon powder suspension diluted to 2-fold with the Tris buffer solution was apportioned thereto, and immediately the side of the microplate was tapped for 10 seconds to mix the contents in the wells and it was allowed to stand at a room temperature for 30 minutes to complete the reaction. On the other hand, each of the anti-IgG-carrying magnetic material-containing latex and anti-IgM-carrying magnetic material-containing latex was diluted 8-fold with the tris buffer solution. They were respectivly apportioned with an amount of 50 µl to either one of the wells of each dilution. The microplate was then tapped as described above to mix the contents and allowed to stand at room temperature for 10 minutes to complete the reaction. Then, by means of small sized rod magnets of 3 mm φ a magnetic field was applied to four sides walls of each well of the microplate for 10 minutes to gather the magnetic material-containing latex to the side portions of the walls. Turbidity of the antigen-carrying carbon powder suspension which remained in the each well without reacting with the magnetic material-containing latex was determined with a Microplate Reader (produced by Nippon Intermed Co., NJ-2000), using a light of a wavelength of 620 nm.

The results are shown in Table 1.

**Table 1**

| | Negative sera | | Positive sera | |
|---|---|---|---|---|
| Dilution fold of sample | Anti-IgG antibody | Anti-IgM antibody | Anti-IgG antibody | Anti-IgM antibody |
| Control | (0.371) | (0.379) | --- | --- |
| 20 | 0.358 | 0.345 | 0.141 | 0.031 |
| 40 | 0.367 | 0.342 | 0.198 | 0.103 |
| 80 | 0.363 | 0.351 | 0.241 | 0.193 |
| 160 | 0.367 | 0.354 | 0.279 | 0.250 |
| 320 | 0.361 | 0.351 | 0.312 | 0.270 |

Both of the anti-IgG or IgM antibody-carried magnetic material-containing latex were reacted with the diluted positive sera sample even when it is diluted to 320-fold, which is markedly superior to the sensitivity of the RPR method. On the other hand, it did not react with the negative sample with any fold dilution.

### Example 2

### Determination of rheumatoid factor

A rheumatoid factor is an anti-human IgG autoantibody found in a serum of a patient suffering from rheumatoid arthritis and is often cross-reacted with other animal IgG's, particularly, rabbit IgG. The immunogloblin class of the rheumatoid factor is usually IgM, but can be IgG and IgA. The relationship between the immunoglobulin class and cause of the disease has attracted attention.

### Preparation of reagents:

### 1) Preparation of a latex reagent carrying rabbit IgG thereon:

In 10 ml of Tris buffer solution was dissolved 4 mg of rabbit γ-globulin (Fr II, containing 70 to 90 % of IgG), and the solution was mixed by stirring for 30 minutes with a suspension of polyvinyltoluene latex (produced by Ceragen Co. ,particle size : 2.02 µm in diameter) in the Tris buffer solution to carry the rabbit γ-globulin on the surface of the latex particles. After centrifugation (10000 rpm, 10 minutes), the supernatant was removed and 20 ml of Tris buffer solution containing 0.3 % bovine serum albumin was added thereto followed by stirring for 30 minutes to re-disperse it. The dispersion was subjected further to an ultrasonic wave treatment to highly disperse and stabilize the latex. Subsequently, after centrifugation, it was dispersed in 20 ml of tris buffer solution containing 0.05 % of sodium azide and preserved at 4 to 10 °C.

### 2) Preparation of a magnetic material-containing latex reagent carrying anti-IgG, IgA or IgM antibody F(ab')₂ thereon

An antiserum obtained by immunizing rabbits against human IgG, human IgA or human IgM was adsorbed onto a Bence Jones protein (χ and λ) to separete a specific antiserum to H-chain of IgG, IgA and IgM. Each of anti-IgG, IgA and Ig M fractions was collected from the resultant antiserum by a conventional method, and then digested with pepsin and subjected to a molecular sieve column chromatography to obtain F(ab')₂.

On the other hand, 1 ml of a magnetic material-containing latex (Estapor® SML266, particle size : 0.7 µm in diameter, 10 %) produced by Rhône Poulenc Chimie was sufficiently mixed with 19 ml of distilled water followed by centrifugation (10000 rpm, 10 minutes), and then the supernatant was removed to obtain a washed latex pellet. To the pellet was added and dispersed an antibody solution in which was dissolved 4 mg of anti-IgG or anti-IgM antibody F(ab')₂ previously prepared in 10 ml of Tris buffer. The dispersion was further stirred for one hour to carry F(ab')₂ on the surface of the magnetic material-containing latex. The dispersion was centrifuged again to remove the supernatant followed by re-dispersing, suspending and stabilizing with 10 ml of the Tris buffer containing 0.3 % of bovine serum albumin.
The resultant latex was further centrifuged and suspended in 10 ml of Tris buffer containing 0.05 % of sodium azide, and preserved at 4 to 10 °C.

### Procedure of the determination:

### 1) Comparison with RAHA method;

Serum samples of twenty rheumatoid patients, which had titers of which were preliminary estimated by the RAHA method (a method of detecting rheumatoid factor by inspecting the occurance of agglutination of sheep erythrocytes sensitized with rabbit IgG), were diluted to 100-fold by using 0.1 % bovine serum albumin and TBS buffer solution. Each of them was apportioned to 3 wells in a microplate in an amount of 100 µl for detecting each class of IgG, IgA and IgM. Thereafter, 50 µl of the rabbit γ-globulin-carrying latex suspension diluted to 8-fold with the Tris buffer solution was apportioned to each well, and immediately the side of the microplate was tapped for 10 seconds to mix the contents. The microplate was allowed to stand at a room temperature for 10 minutes to complete the reaction. On the other hand, each of the anti-IgG, anti-IgA and anti-IgM-carrying latex reagents was diluted 8-fold with the Tris buffer solution. They were apportioned in an amount of 50 µl to one of the three wells, respectively. Thereafter, the microplate was tapped as described above to mix the contents and allowed to stand at room temperature for 10 minutes to complete the reaction. Then, by means of small sized rod magnets of 3 mm φ a magnetic field was applied to four side walls of each well of the microplate for 10 minutes to gather the magnetic material-containing latex to the side walls. Turbidity of the rabbit γ-globulin-carrying latex suspension which remained in each of the wells without reacting with the magnetic material-containing latex was determined with a Microplate Reader (produced by Nippon Intermed Co., NJ-2000) using a light of a wavelength of 620 nm. The experimental results of this example were compared with the titers detected by the RAHA method. The results are shown in Table 2.

**Table 2**

| Sample No. | Title by PAHA method | Data obtained by the present method | | |
|---|---|---|---|---|
| | | IgG | IgA | IgM |
| Control | | 0.490 | 0.502 | 0.526 |
| 3 | < 20 | 0.525 | 0.518 | 0.432 |
| 8 | < 20 | 0.529 | 0.513 | 0.336 |
| 10 | < 20 | 0.526 | 0.525 | 0.514 |
| 12 | 20 | 0.539 | 0.540 | 0.542 |
| 20 | 20 | 0.568 | 0.529 | 0.518 |
| 25 | 40 | 0.538 | 0.477 | 0.497 |
| 26 | 640 < | 0.369 | 0.479 | 0.182 |
| 30 | 40 | 0.535 | 0.561 | 0.420 |
| 31 | 20 | 0.533 | 0.614 | 0.373 |
| 41 | 640 < | 0.232 | 0.492 | 0.152 |
| 48 | 320 | 0.342 | 0.157 | 0.311 |
| 52 | 320 | 0.561 | 0.539 | 0.300 |
| 55 | 640 < | 0.286 | 0.263 | 0.112 |
| 56 | 640 < | 0.472 | 0.576 | 0.186 |
| 89 | 640 < | 0.431 | 0.443 | 0.163 |
| 90 | 640 < | 0.393 | 0.237 | 0.134 |
| 173 | 640 < | 0.225 | 0.175 | 0.127 |
| 400 | 640 < | 0.214 | 0.108 | 0.128 |
| 459 | 640 < | 0.218 | 0.132 | 0.142 |
| 535 | 640 < | 0.515 | 0.406 | 0.159 |

There is a tendency that the specimens having a higher titer in the RAHA method almost show relatively high reactivity, and the relationship of the rheumatoid factor with the IgM class is particularly high.

### 2) Comparison with EIA method

One sample which had been proved, according to the results of the foregoing determination (1), to contain every class of rheumatoid factors of IgG, IgA and IgM, was selected and diluted with the TBS buffer from 60-fold to 131220-fold stepwise by a 3-fold dilution. A series of these dilutions were completed in the same manner as in the foregoing procedure (1). The results were compared to those of the EIA method.

The EIA method was conducted as follows:
Anti-human IgG, IgA and IgM F(ab')₂ which are the same as those carried on the magnetic material-containing latex reagent were labelled with peroxidase according to the method of Mukoujima, "Japan Clinics", Vol. 37, Summer season special issue, p. 112 (1979)). A solution of 100 µg/ml of rabbit γ-globulin in PBS buffer was adsorbed to a microplate followed by undergoing a treatment with bovine serum albumin-PBS buffer solution to prepare a solid phase. The same samples as described above were diluted from 20-fold to 43740-fold stepwise by a 3-fold dilution. Each of these diluted samples was apportioned to a 50 µl well and reacted at 37 °C for 2 hours. Subsequently, after the plate was washed five times with the PBS buffer solution, each of the 50 µl of the above labelled antibody diluted from 1000 to 2000-fold was apportioned thereto and reacted at 37 °C for 2 hours. After washing with the PBS buffer solution ten times, the resultant was reacted with 4-aminoantipyrine as a color producing agent, at room temperature for 30 minutes , and an estimation was carried out with a Microplate Reader, using a light of a wavelength of 490 nm.

The results are shown in Figs. 1, 2 and 3.

In the EIA method, when the reactivity is high, the absorbance increases. This means that the higher the dilution fold of the sample becomes, the more the absorbance decreases. In contrast, according to the present invention, the reactivity can be estimated as a degree of decrease in turbidity. This means that the higher the dilution fold of the sample becomes, the lower the reactivity becomes, the more the turbidity increases.

The maximum absorbance in each of the EIA and the present method is about 0.6. It corresponds to the reactivity of 50 %. In the sample of the dilution-fold corresponding to the absorbance of 0.3, the method of the present invention is excellent in detection sensitivity as compared with the EIA, i.e., 5-fold in IgG, 90-fold in IgA and 12-fold in IgM, respectively.

## Claims

1. A method of determination and detection of an antibody in a biological fluid sample against a specific antigen and of its immunoglobulin class, comprising the step of:
(a) providing a first reagent of insoluble substrate particles carrying the antigen on the surface thereof and a second reagent of insoluble substrate particles carrying on the surface thereof a substance particularly reactive to a specific immunoglobulin class, the latter reagent containing a magnetic material;
(b) reacting the sample with both of the reagents in a reaction system;
(c) agglomerating the antigen-carrying particles together with the magnetic material-containing particles through the antibody;
(d) separating locally in the reaction system the unreacted magnetic material-containing particles and the resultant agglomerate by applying a magnetic field to the reaction system; and
(e) visually or spectrophotometrically estimating the amount of the antigen-carrying particles which remain unreacted in the reaction system.

2. The method of Claim 1, wherein said insoluble substrate particles as the first reagent are composed of cells, gelatin particles, microcapsules, organic polymers, inorganic fine particles or colloidal particles of metal or a metal compound.

3. The method of Claim 1 or 2, wherein said organic polymer is polyvinyltoluene or polystyrene.

4. The method according to any one of Claims 1 to 3, wherein said inorganic fine particle is carbon black.

5. The method according to any one of Claims 1 to 4, wherein the substance which is particularly reactive to the specific immunoglobulin class is an antibody against IgG, IgA, IgM, IgD, IgE or L-chains thereof, protein A or C_{1q}.

6. The method according to any one of Claims 1 to 5, wherein said magnetic material-containing particles are composed of polysaccarides, proteins, protein derivatives or synthetic polymers.

7. The method of Claim 6, wherein said synthetic polymer is a polymer obtained by polymerizing an aromatic vinyl compound and/or methacrylate derivative.

8. The method according to any one of Claims 1 to 5, wherein the magnetic material contained in said second reagent is iron or a tri-iron tetroxide.

9. The method according to any one of Claims 1 to 5, wherein the magnetic material has an average particle size of 1 to 20 nm (10 to 200 Å) in diameter.

10. The method according to any one of Claims 1 to 5, wherein the content of the magnetic material is at least 5% by weight of the magnetic material-containing particles.

11. The method of Claim 10, wherein the content of the magnetic material is of 15 to 65 % by weight of the magnetic material-containing particles.

12. The method according to any one of Claims 1 to 4, wherein the average particle size of the first insoluble substrate particles and the second magnetic material-containing particles is of 0.1 to 10 µm in diameter.

13. The method of Claim 12, wherein the average particle size is of 0.2 to 3 µm in diameter.

14. The method according to any one of Claims 1 to 4, wherein the average particle size of the insoluble substrate particles as the first reagent is of 0.5 to 3 µm in diameter and that of the magnetic material-containing particles as the second reagent is of 0.2 to 2 µm in diameter.

15. The method of Claim 14, wherein the average particle size of the insoluble substrate particles as the first reagent is of 1 to 2.5 µm in diameter and that of the magnetic material-containing particles as the second reagent is of 0.5 to 1.5 µm in diameter.

16. The method according to any one of Claims 1 to 15, wherein the ratio of the first insoluble substrate particles to the second magnetic material-containing particle to be participated in the reaction is of from 1 : 20 to 20 : 1, preferably from 1 : 4 to 4 : 1.

17. The method according to any one of Claims 1 to 16, wherein the average particle size of the first insoluble substrate particles is of 1 to 2.5 µm in diameter and that of the second magnetic material-containing particles is of 0.5 to 1.5 µm in diameter, and the ratio of the former particles to the latter particles is of from 1 : 0.8 to 1 : 2.

## Patentansprüche

1. Verfahren zur Bestimmung und zum Nachweis eines Antikörpers gegen ein spezifisches Antigen und seiner Immunglobulin-Klasse in einer Probe einer biologischen Flüssigkeit, welches folgende Stufen umfaßt:
(a) Bereitstellen eines ersten Reagenz aus unlöslichen Substratteilchen, die auf ihrer Oberfläche das Antigen tragen, und eines zweiten Reagenz aus unlöslichen Substratteilchen, die auf ihrer Oberfläche eine Substanz tragen, die speziell reaktiv gegenüber einer spezifischen Immunglobulin-Klasse ist, wobei das letztere Reagenz ein magnetisches Material enthält,
(b) Umsetzen der Probe mit beiden Reagenzien in einem Reaktionssystem,
(c) Agglomerieren der das Antigen tragenden Teilchen zusammen mit den das magnetische Material enthaltenden Teilchen mittels des Antikörpers,
(d) örtliche Trennung der nicht umgesetzten, magnetisches Material enthaltenden Teilchen und des gebildeten Agglomerats in dem Reaktionssystem durch Anlegen eines Magnetfeldes an das Reaktionssystem und
(e) visuelle oder spektrophotometrische Bestimmung der Menge der in dem Reaktionssystem nicht umgesetzt verbliebenen, das Antigen tragenden Teilchen.

2. Verfahren nach Anspruch 1, wobei die das erste Reagenz darstellenden unlöslichen Substratteilchen aus Zellen, Gelantineteilchen, Mikrokapseln, organischen Polymeren, anorganischen feinen Teilchen oder kolloidalen Teilchen aus einem Metall oder einer Metallverbindung bestehen.

3. Verfahren nach Anspruch 1 oder 2, wobei das organische Polymere Polyvinyltoluol oder Polystyrol ist.

4. Verfahren nach einem der Ansprüche 1 bis 3, wobei die anorganischen feinen Teilchen aus Ruß bestehen.

5. Verfahren nach einem der Ansprüche 1 bis 4, wobei die Substanz, die speziell reaktiv gegenüber der spezifischen Immunglobulin-Klasse ist, ein Antikörper gegen IgG, IgA, IgM, IgD, IgE oder deren L-Ketten, Protein A oder C_{1q} ist.

6. Verfahren nach einem der Ansprüche 1 bis 5, wobei die ein magnetisches Material enthaltenden Teilchen aus Polysacchariden, Proteinen, Proteinderivaten oder synthetischen Polymeren bestehen.

7. Verfahren nach Anspruch 6, wobei das synthetische Polymere ein durch Polymerisation einer aromatischen Vinylverbindung und/oder eines Methacrylat-Derivats erhaltenes Polymeres ist.

8. Verfahren nach einem der Ansprüche 1 bis 5, wobei das in dem zweiten Reagenz enthaltene magnetische Material Eisen oder ein Trieisentetroxid ist.

9. Verfahren nach einem der Ansprüche 1 bis 5, wobei das magnetische Material eine durchschnittliche Teilchengröße mit einem Durchmesser von 1 bis 20 nm (10 bis 200 Å) hat.

10. Verfahren nach einem der Ansprüche 1 bis 5, wobei der Gehalt an magnetischem Material mindestens 5 Gew.-% der das magnetische Material enthaltenden Teilchen beträgt.

11. Verfahren nach Anspruch 10, wobei der Gehalt an magnetischem Material 15 bis 65 Gew.-% der das magnetische Material enthaltenden Teilchen beträgt.

12. Verfahren nach einem der Ansprüche 1 bis 4, wobei die durchschnittliche Teilchengröße der ersten unlöslichen Substratteilchen und der zweiten das magnetische Material enthaltenden Teilchen 0,1 bis 10 µm, als Durchmesser, beträgt.

13. Verfahren nach Anspruch 12, wobei die durchschnittliche Teilchengröße 0,2 bis 3 µm, als Durchmesser, beträgt.

14. Verfahren nach einem der Ansprüche 1 bis 4, wobei die durchschnittliche Teilchengröße der das erste Reagenz darstellenden unlöslichen Substratteilchen 0,5 bis 3 µm als Durchmesser, und die der das zweite Reagenz darstellenden das magnetische Material enthaltenden Teilchen 0,2 bis 2 µm, als Durchmesser, beträgt.

15. Verfahren nach Anspruch 14, wobei die durchschnittliche Teilchengröße der das erste Reagenz darstellenden unlöslichen Substratteilchen 1 bis 2,5 µm, als Durchmesser und die der das zweite Reagenz darstellenden das magnetische Material enthaltenden Teilchen 0,5 bis 1,5 µm, als Durchmesser, beträgt.

16. Verfahren nach einem der Ansprüche 1 bis 15, wobei das Verhältnis der ersten unlöslichen Substratteilchen zu dem zweiten das magnetische Material enthaltenden Teilchen, die an der Reaktion teilnehmen, 1:2 bis 20:1, vorzugsweise 1:4 bis 4:1 beträgt.

17. Verfahren nach einem der Ansprüche 1 bis 16, wobei die durchschnittliche Teilchengröße der ersten unlöslichen Substratteilchen 1 bis 2,5 µm, als Durchmesser, und die der zweiten das magnetische Material enthaltenden Teilchen 0,5 bis 1,5 µm, als Durchmesser, beträgt und das Verhältnis der ersteren Teilchen zu den letzteren Teilchen 1:0,8 bis 1:2 beträgt.

## Revendications

1. Procédé pour la détermination et la détection d'un anticorps d'un antigène spécifique et de sa classe d'immunoglobuline dans un échantillon de fluide biologique, comprenant les étapes de :
(a) obtention d'un premier réactif constitué de particules d'un substrat insoluble portant l'antigène sur leur surface et d'un second réactif constitué de particules d'un substrat insoluble portant sur leur surface une substance particulièrement réactive par rapport à une classe spécifique d'immunoglobulines, le second réactif contenant un matériau magnétique ;
(b) réaction de l' échantillon avec les deux réactifs dans un système réactionnel;
(c) agglomération des particules portant l'antigène avec les particules contenant le matériau magnétique par l'intermédiaire de l'anticorps ;
(d) séparation localement dans le système réactionnel des particules contenant le matériau magnétique n'ayant pas réagi et de l'agglomérat résultant en appliquant un champ magnétique au système réactionnel ; et
(e) estimation visuelle ou spectrophotométrique de la quantité de particules portant l'antigène qui n'ont pas réagi restant dans le système.

2. Procédé selon la revendication 1, dans lequel les dites particules de substrat insoluble constituant le premier réactif sont composées de cellules, de particules de gélatine, de microcapsules, de polymères organiques, de fines particules inorganiques ou de particules colloïdales de métal ou de composé métallique.

3. Procédé selon l'une des revendications 1 ou 2, dans lequel ledit polymère organique est un polyvinyltoluène ou un polystyrène.

4. Procédé selon l'une quelconque des revendications 1 à 3, dans lequel les dites particules inorganiques fines sont du noir de carbone.

5. Procédé selon l'une quelconque des revendications 1 à 4, dans lequel la substance qui est particulièrement réactive par rapport à une classe spécifique d'immunoglobuline est un anticorps de IgG, IgA, IgM, IgD, IgE ou leurs chaînes L, la protéine A ou C_{1q}.

6. Procédé selon l'une quelconque des revendications 1 à 5, dans lequel les dites particules contenant un matériau magnétique sont composées de polysaccharides, de protéines, de dérivés de protéine ou de polymères synthétiques.

7. Procédé selon la revendication 6, dans lequel le dit polymère synthétique est un polymère obtenu par polymérisation d'un composé vinyl aromatique et/ou d'un dérivé méthacrylate.

8. Procédé selon l'une quelconque des revendications 1 à 5, dans lequel le matériau magnétique contenu dans le dit second réactif est du fer ou Fe₃O₄.

9. Procédé selon l'une quelconque des revendications 1 à 5, dans lequel le matériau magnétique a une dimension moyenne de particules de 1 à 20 nm (10 à 200 Å) en diamètre.

10. Procédé selon l'une quelconque des revendications 1 à 5, dans lequel la teneur en matériau magnétique est d'au moins 5 % en poids par rapport aux particules contenant le matériau magnétique.

11. Procédé selon la revendication 10, dans lequel la teneur en matériau magnétique est 15 à 65 % en poids par rapport aux particules contenant le matériau magnétique.

12. Procédé selon l'une quelconque des revendications 1 à 4, dans lequel la dimension moyenne de particule des premières particules de substrat insoluble et des secondes particules contenant un matériau magnétique est de 0,1 à 10 µm en diamètre.

13. Procédé selon la revendication 12, dans lequel la dimension moyenne de particule est de 0,2 à 3 µm en diamètre.

14. Procédé selon l'une quelconque des revendications 1 à 4, dans lequel la dimension moyenne de particule des particules de substrat insoluble constituant le premier réactif est de 0,5 à 3 µm en diamètre et celui des particules contenant un matériau magnétique constituant le second réactif est de 0,2 à 2 µm en diamètre.

15. Procédé selon la revendication 14, dans lequel la dimension moyenne de particule des particules de substrat insoluble constituant le premier réactif est de 1 à 2,5 µm en diamètre et celui des particules contenant un matériau magnétique constituant le second réactif est de 0,5 à 1,5 µm en diamètre.

16. Procédé selon l'une quelconque des revendications 1 à 15, dans lequel le rapport entre les premières particules de substrat insoluble et les secondes particules contenant le matériau magnétique impliquées dans la réaction est de 1/20 à 20/1, de préférence de 1/4 à 4/1.

17. Procédé selon l'une quelconque des revendications 1 à 16, dans lequel la dimension moyenne de particule des premières particules de substrat insoluble est de 1 à 2,5 µm en diamètre et celui des secondes particules contenant un matériau magnétique est de 0,5 à 1,5 µm en diamètre, et le rapport entre les premières particules et les secondes particules est de 1/0,8 à 1/2.
